# EUROPÄISCHE PATENTSCHRIFT

(11) **EP 1 791 881 B1**
(45) Veröffentlichungstag und Bekanntmachung des Hinweises auf die Patenterteilung: **26.03.2008**
(21) Anmeldenummer: 05794148.6
(22) Anmeldetag: 22.09.2005
(51) Int. Cl.: C08G 18/32, C08G 18/64, C08K 5/1545, A61L 27/38, A61L 27/50, A61L 27/56

(54) **OFFENPORIGER POLYURETHANSCHAUM OHNE HAUTBILDUNG, FORMULIERUNG ZU SEINER HERSTELLUNG UND VERWENDUNG DESSELBEN ALS TRÄGERMATERIAL FÜR ZELL- UND GEWEBEKULTUREN ODER ARZNEIMITTEL**
OPEN-PORED POLYURETHANE FOAM WITHOUT SKIN FORMATION, FORMULATION FOR THE PRODUCTION THEREOF AND USE THEREOF AS A CARRIER MATERIAL FOR CELL AND TISSUE CULTURES OR MEDICAMENTS
MOUSSE POLYURETHANNE A PORES OUVERTS EXEMPTE DE PEAU, FORMULATION SERVANT A PRODUIRE CETTE MOUSSE POLYURETHANNE, ET UTILISATION DE LADITE MOUSSE POLYURETHANNE EN TANT QUE MATIERE DE SUPPORT POUR LA CULTURE CELLULAIRE OU LA CULTURE TISSULAIRE, OU EN TANT QUE MEDICAMENT

(30) Priorität: 23.09.2004 DE 102004046172
(43) Veröffentlichungstag der Anmeldung: 06.06.2007
(73) Patentinhaber: Polymaterials AG, 87600 Kaufbeuren (DE)
(72) Erfinder: WIESE, Hinrich, 86899 Landsberg am Lech (DE); MAIER, Gerhard, 80807 München (DE)
(74) Vertreter: Adam, Holger
(86) Internationale Anmeldenummer: PCT/EP2005/010266
(87) Internationale Veröffentlichungsnummer: WO 2006/032501

(56) Entgegenhaltungen:
- EP-A- 0 913 415
- WO-A-20/04060948
- DE-A1- 19 544 121
- US-B1- 6 376 742
- GORNA KATARZYNA ET AL: "Preparation, degradation, and calcification of biodegradable polyurethane foams for bone graft substitutes" MEDLINE, 2003, XP002319789

## Beschreibung

Die vorliegende Erfindung betrifft eine Formulierung zur Herstellung eines biokompatiblen, gegebenenfalls bioabbaubaren offenporigen Polyurethanschaums, der ohne mechanische Nachbehandlung auch an seiner Oberfläche geöffnete Poren besitzt, also keine Haut aufweist, einen derartigen Polyurethanschaum sowie ein Verfahren zu seiner Herstellung und seine Verwendung. Die Größe der Poren des durch Erwärmen der Formulierung erhaltenen Polyurethanschaums nimmt dabei in den oberflächennahen Bereichen des Schaums zu, ebenso die Anzahl der Poren, die zur Außenseite des Schaums hin offen sind. Dies macht den erfindungsgemäßen Polyurethanschaum zur Verwendung bei der Herstellung eines Zellträgers (Scaffold) für Zellkulturen, Gewebekulturen und für das Tissue Engineering besonders geeignet. Des Weiteren betrifft die vorliegende Erfindung also ein Verfahren zur Herstellung eines Zellträgers für Zellkulturen, Gewebekulturen und für das Tissue Engineering, das die Formulierung zur Herstellung des offenporigen Polyurethanschaums verwendet, sowie einen durch dieses Verfahren erhaltenen Zellträger für Zellkulturen, Gewebekulturen und für das Tissue Engineering.

Geschäumte Polymere finden in den verschiedensten Bereichen Anwendung. Sie sind nicht nur leichter als kompakte Materialien sondern weisen noch verschiedene weitere Vorteile auf. Man unterscheidet hierbei zunächst nach der Materialbasis und dann danach, ob die Poren miteinander verbunden sind (offenporiger Schaum oder Schwamm) oder ob sie durch unterschiedlich kompakte Wandungen getrennt sind (geschlossenporiger Schaum).

Offenporige Polyurethanschäume oder -schwämme werden in den verschiedensten Gebieten, von Putzschwämmen über Filtermaterialien bis zu Scaffolds (Kunststoffgerüste als Träger für Zellen) für das Tissue Engineering (Geweberegeneration), eingesetzt. Die wirtschaftliche Bedeutung solcher Schwämme ist sehr hoch, da in einigen Bereichen, wie der Automobilindustrie sehr große Mengen benötigt werden, in anderen, wie der Medizintechnik, die Materialien sehr hochwertig sind. Der Bereich der Schaumherstellung und Verbesserung ist daher permanenter Gegenstand intensiver Forschung.

So gibt es verschiedene Verfahren, Polyurethane in Form von Schäumen oder Schwämmen herzustellen (A. J. DeVries, Rubber Chem. Technol. 1958, 31, 1142):
- Durch unter Druck gelöste inerte Gase
- Durch Verdampfen leichtflüchtiger inerter Flüssigkeiten
- Durch Verbindungen, die sich bei erhöhter Temperatur zu Gasen zersetzen (reversibel wie Alkali- und Erdalkalicarbonate oder irreversibel wie Azo- und Diazoverbindungen)
- Durch Wasser, das mit Isocyanaten unter Bildung von CO₂ reagiert
- Durch Füllstoffe, die nach dem Härten herausgelöst werden (z. B. wasserlösliche Salze)
- Durch Polymerisation in Lösung unter Phasentrennung (Lösemittel löst die Edukte, aber nicht das Polymer, F. D. Hileman, R. E. Sievers, G. G. Hess, W. D. Ross, Anal. Chem. 1973, 45, 1126-1130)
- Durch mechanisches Einmischen von Gasen (Luft), z.B. durch Rühren

Die Offenporigkeit kann hierbei durch Additive, besonders Tenside (vgl. Mattesky in US 6 391 933), unterstützt werden. Bei physikalischen Schäumungsmitteln sind auch noch Nukleierungsmittel notwendig oder zumindest vorteilhaft, um gleichmäßig große und gleichmäßig verteilte Poren zu erhalten.

Die Herstellung von Polymerschäumen kann entweder durch Aufschäumen eines bereits vorgebildeten Polymers erfolgen oder aus Monomeren durch gleichzeitige Polymerisation und Schaumbildung. In allen Fällen beruht die Schaumbildung darauf, dass in eine flüssige Zubereitung, die z.B. aus einer Polymerschmelze, einer Polymerlösung, einer Polymerdispersion oder einer Monomermischung aus einer oder mehreren Komponenten besteht, ein Treibmittel eingebracht wird, welches unter den Schäumbedingungen gasförmig ist. Die Schaumbildung wird ausgelöst, indem die Konzentration des gasförmigen Treibmittels in der flüssigen Zubereitung über seine Sättigungskonzentration gebracht wird. Dies kann z.B. dadurch geschehen, dass die Temperatur erhöht oder der Druck gesenkt wird, oder aber auch dadurch, dass durch Auslösen einer chemischen Reaktion eine hinreichend große Menge an Treibmittel (Gas) erzeugt wird. Der letztere Fall wird häufig bei der Herstellung von Polyurethanschäumen genutzt. Überschreitet die Gaskonzentration die Sättigungskonzentration, so kommt es in einer ersten Phase zur Blasenbildung, wenn Nukleierungsmittel vorhanden sind. Häufig kann ein befriedigendes Ergebnis auch ohne Nukleierungsmittel erreicht werden, da in den meisten Fällen insbesondere bei Systemen, die gleichzeitig polymerisieren und schäumen, bereits durch die Vermischung der Komponenten genügend Mikrobläschen vorhanden sind. In der zweiten Phase der Schaumbildung wachsen die Blasen, wobei aufgrund des Druckunterschieds in Blasen unterschiedlicher Größe die großen Blasen auf Kosten der kleinen wachsen. In dieser Phase müssen Schäumgeschwindigkeit, Oberflächenspannung und Viskosität bzw. Viskositätsänderung des Polymers exakt aufeinander abgestimmt werden, damit die Blasen erhalten bleiben und das Treibmittel nicht vorzeitig entweicht, da dies zum Kollaps des Schaums führen würde. In der nächsten Phase erfolgt die Verfestigung des Polymers z.B. durch Polymerisation, Vernetzung oder Abkühlung, so dass der Schaum seine endgültige Ausdehnung erreicht. In Abhängigkeit vom Innendruck der Blasen (Poren oder Zellen) und den mechanischen Eigenschaften des Polymers kann es in dieser Phase zu Veränderungen der makroskopischen Ausdehnung des Schaums kommen.

Während des Wachstums der einzelnen Blasen werden die Flüssigkeits- bzw. Polymerfilme zwischen den einzelnen Blasen immer dünner, da das Gesamtvolumen des Schaums stark ansteigt. Der größte Teil des Materials konzentriert sich auf die Stege, welche die Zwickel zwischen den einzelnen Blasen verbinden. Kommt es in dieser Phase zu häufigen Rissen der dünnen Filme zwischen den Blasen, so kann das Treibmittel vorzeitig entweichen und der Schaum kollabiert oder es bilden sich unkontrolliert große Hohlräume. Bleiben die Blasen bis zur Verfestigung des Polymers weit überwiegend erhalten, so spricht man von einem Schaum mit geschlossenen Poren. In den Poren befindet sich dann das Treibmittel, das über einen mehr oder weniger langen Zeitraum durch Diffusion verloren geht und durch Luft ersetzt wird. Solche Schäume eignen sich beispielsweise bevorzugt zur Schall- und Wärmeisolation.

Offenporige Schäume zeichnen sich dagegen dadurch aus, dass die weit überwiegende Zahl der Bläschen zu mindestens zwei benachbarten Bläschen offen ist, der Polymerfilm dazwischen also gerissen oder nicht mehr vorhanden ist. Hier kann zwischen den Poren ein freier Austausch von Gasen oder Flüssigkeiten erfolgen. Solche Materialien eigenen sich bevorzugt als Filter oder Absorptionsmaterialien, aber auch als Zellträger für Tissue Engineering -(Geweberegeneration) in medizinischen Anwendungen.

Bei Polyurethanschäumen wurde beobachtet (J. H. Saunders, Fundamentals of Foam Formation, in D. Klempner, K. C. Frisch, Handbook of Polymeric Foams and Foam Technology. Hanser, München 1991, S.12), dass offenporige Schäume insbesondere dadurch entstehen, dass gegen Ende des Blasenwachstums, kurz bevor sich das Polymer verfestigt, eine signifikante Steigerung der Gasbildungsgeschwindigkeit, also der Treibmittelmenge, auftritt, welche die Blasen zum "Platzen" bringt. Dabei muss das Polymer aber schon so weit verfestigt sein, dass die Stege zwischen den Zwickeln zwischen den einzelnen Blasen stabil genug sind, um die äußere Form des Schaumkörpers zu erhalten. Es konnte gezeigt werden, dass dies genau dann auftritt, wenn die innere Temperatur des Schaums durch die Wärmetönung der ablaufenden chemischen Reaktion auf 100°C steigt, wobei Wasser aus der ursprünglichen Monomermischung verdampft und der Wasserdampf als zusätzliche Gasmenge zur Verfügung steht. Tatsächlich wurden ca. 20% Wasserdampf in der Gasmischung in den Poren solcher Schäume gefunden.

Geschlossenporige, aber auch offenporige Schäume besitzen zur äußeren Umgebung (Schäumwerkzeug, Atmosphäre etc.) stets eine "Haut", also eine Oberfläche, die keine oder nur wenige Poren enthält. Dies wird bei den sogenannten Integralschäumen gezielt eingestellt und genutzt, um eine mechanisch belastbare, dichte Außenhaut bei gleichzeitig möglichst geringer Dichte und hoher Porosität im Inneren zu erreichen.

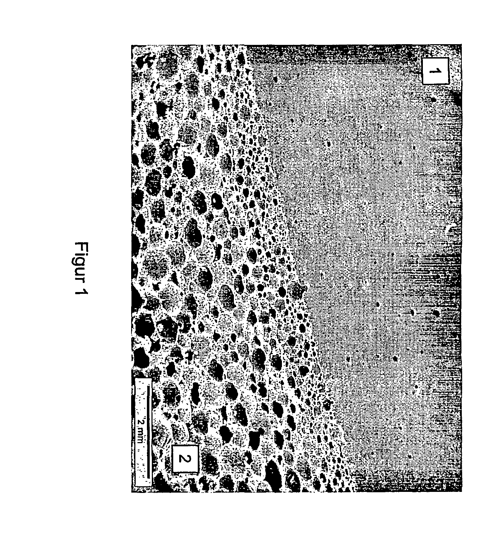

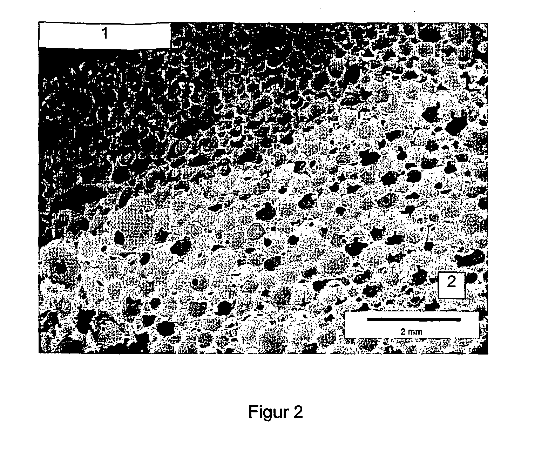

Die Bildung der Haut wird sowohl beim freien Schäumen als auch beim Schäumen in einer geschlossenen Form beobachtet. Ein Beispiel für eine solche Haut bei einem offenporigen Schaum ist z.B. in US 2002/0062097, Fig. 1 und 2, zu sehen. Als Ursachen für die Hautbildung werden mehrere Möglichkeiten diskutiert: der Aufbau eines Druckgradienten, die Oberflächenspannung des Schaums und der Aufbau eines Temperaturgradienten zwischen Schaum und Form bzw. Werkzeug.

Ein Druckgradient kommt zustande, da das Treibmittel nicht durch die Wand der Schäumform bzw. des Werkzeugs ausgasen kann, so dass sich in der Nähe der Wand ein höherer Druck aufbaut als im Inneren des Schaums. Die steigende Viskosität des Materials während des Schäumens verhindert den einfachen Druckausgleich. Da die Porengröße vom Druck abhängt, bilden sich zur Wand hin immer kleinere Poren und letztlich dann die Haut, da direkt an der Form- bzw. Werkzeugwand kein Treibmittel entweichen kann. Hinzu kommt die Oberflächenspannung des Polymers, die dazu führt, dass jede Treibmittelblase bzw. Pore, die sich öffnen soll, eine Energiezufuhr benötigt, da durch das Öffnen der Pore die Oberfläche des Polymers vergrößert wird und die dafür erforderliche Energie aufgebracht werden muss. Diese Effekte werden insbesondere im Fall der Polyurethanschäume und chemisch verwandter Schäume verstärkt durch den Aufbau eines Temperaturgradienten.

Offenporigkeit von Polyurethanschäumen wird technisch beispielsweise durch eine zusätzliche Zufuhr an Gas erreicht, das in Form von Wasserdampf beim Erreichen einer Innentemperatur von 100°C im Inneren des Schaums während des Schäumvorgangs plötzlich gebildet wird, wodurch die Gasblasen bzw. Poren zum Platzen gebracht werden. Wenn aber die Form- oder Werkzeugwand bzw. die Umgebung des Schaums beim freien Schäumen kühler ist, erfolgt der zur Porenöffnung nötige "Treibmittelstoß" nicht in der Nähe der Wand bzw. der Oberfläche, so dass die Poren, die sich dort befinden, nicht geöffnet werden und somit auch die Oberfläche geschlossen bleibt (R. Brathun, P. Zingsheim, PVC Foams, in D. Klempner, K. C. Frisch, Handbook of Polymeric Foams and Foam Technology. Hanser, München 1991, S.246/47).

Für bestimmte Anwendungen z.B. als Filtermedien, Schwamm oder auch als biokompatibler Zellträger für Anwendungen im Tissue Engineering ist es jedoch erforderlich, dass auch die Haut offenporig ist, so dass ein ungehinderter Zugang von außen zu den inneren, offenen, d.h. interkonnektierenden Poren besteht.

Für viele Anwendungen ist es problemlos möglich, diese Haut mechanisch zu entfernen, indem z.B. vorgeschäumte Halbzeuge zurechtgeschnitten werden, wobei die äußeren Bereiche mit der Haut einfach abgeschnitten werden, was mit einem Verlust an Material einhergeht. Im Fall komplexer Geometrien der geschäumten Teile ist dies aber mit einem sehr hohen Aufwand verbunden. Ein Möglichkeit zur Lösung dieses Problems besteht darin, dass nach dem Einspitzen der reaktiven Masse in die Form zu einem bestimmten Zeitpunkt während des Schäumvorgangs der Druck verringert oder sogar Unterdruck angelegt wird (K. D. Cavender, J. Cell. Plast. 1986, 22, 222-234 und K. D. Cavender, in US 4579700*,* Union Carbide Corp., USA, **1986**). Aber dieses Verfahren kann nicht in allen Fällen angewandt werden (z. B. abhängig von der Auslegung der Form) und führt zudem meist zu relativ großen, gerichteten Poren und nicht dazu, dass sich die Poren einfach ohne Hautbildung bis zur Werkzeugwand fortsetzen.

Der Einsatz eines inerten Füllstoffs (z. B. Salz) zusätzlich zur Porengenerierung durch Gas und sein anschließendes Herauslösen, wie dies gelegentlich bei Schäumen, die als Zellträger für Tissue Engineering eingesetzt werden sollen, angewendet wird, führt ebenfalls dazu, dass einige Poren die Haut durchdringen, nämlich an den Stellen, an denen sich der inerte Füllstoff an der Oberfläche befunden hat und nachträglich herausgelöst wurde. Dies erfordert jedoch einen weiteren Verfahrensschritt und kann zudem dazu führen, dass unerwünschte Reste des Füllstoffs im Polymer verbleiben, was insbesondere für medizinische Anwendungen wie dem Tissue Engineering mit großen Nachteilen verbunden ist.

Tissue Engineering ist ein Bereich der Anwendungen für neue Materialien mit sehr großem Wachstumspotential: Bei Gewebe- und Organdefekten, bedingt durch Trauma, Krankheit oder angeborene Missbildung, stoßen die herkömmlichen Therapien wie die Implantation von Prothesen (natürlich wie Spenderknochen oder -gewebe oder künstlich hergestellt wie Metallimplantate, Kunststoffimplantate) immer mehr an ihre Grenzen (Infektionen, Abstoßungsreaktionen des Gewebes). Zudem ist die herkömmliche Implantation von Prothesen im Bereich des Bindegewebes häufig durch die eingeschränkte Funktionsfähigkeit und Dauerbelastbarkeit künstlicher Materialien gekennzeichnet [C. W. Patrick, A. G. Mikos, L. V. Mclntire, (Hrsgb.) Frontiers of Tissue Engineering, Elsevier Science Ltd. Oxford 1998]. Darum ist die moderne Medizin bestrebt, autologe Implantate (Implantate, die aus patienteneigenen Zellen bzw. Gewebe auf Zellträgern aus biokompatiblen, resorbierbaren Materialien wie z.B. synthetischen Polymeren bestehen) herzustellen. Einer der erfolgverheißendsten Ansätze seit Anfang der 90er Jahre ist hier das Tissue Engineering durch Züchtung von Zellen aus Eigenspende des Empfängers auf einem porösen Polymergerüst, das dann biologisch unter Bildung unschädlicher Produkte abgebaut wird. Das Ziel sind funktionsfähige Ersatzgewebe, deren Form genau zum lmplantationsort bzw. dem zu heilenden Defekt passt und vom Empfänger nicht abgestoßen werden, da sie aus seinen eigenen Zellen gebildet sind. Neben verschiedenen Polymeren, deren mechanische Eigenschaften weit von denen des zu bildenden Gewebes entfernt sind (Polylactid, Polyglycolid, Alginate, Fibrinkleber), kommen vor allem auch Polyurethane in Frage. Polyurethane bieten den Vorteil, daß ihre mechanischen Eigenschaften in einem weiten Bereich modifiziert werden können, der auch die vieler körpereigener Gewebe umfasst (z. B. Knorpel, Adern, Sehnen). Über die Bausteine der Polyurethane ist auch die Geschwindigkeit des Abbaus einstellbar [N. M. K. Lamba, K. A. Woodhouse, S. L. Cooper, Polyurethanes in Biomedical Applications, CRC Press, Boca Raton, Boston, London, New York, Washington, 1998].

Der vorliegenden Erfindung liegt also die Aufgabe zugrunde, eine Formulierung bzw. Zusammensetzung zur Herstellung eines offenporigen Polyurethanschaums zur Verfügung zustellen, der auch an seiner Oberfläche eine Vielzahl geöffneter Poren besitzt, also keine Haut aufweist, und der die oben beschriebenen, im Stand der Technik auftretenden Probleme überwindet. Insbesondere soll der aus der Formulierung resultierende Polyurethanschaum biokompatibel sein und die Anzahl und Größe der Poren soll wenigstens über den ganzen Polyurethanschaum homogen verteilt sein oder bevorzugter in den oberflächennahen Bereichen des Schaums zunehmen, wodurch der Polyurethanschaum für Zellkulturen, Gewebekulturen und für das Tissue Engineering geeignet wird.

Überraschend wurde nun bei der Erprobung von Additiven für Polyurethanformulierungen für das Tissue Engineering gefunden, dass der Einsatz von bestimmten Mono-, Di-, Oligo- und Polysacchariden in solchen Formulierungen in kleinen Mengen dazu führt, dass die Porosität sowie die Offenporigkeit deutlich verbessert werden können, so dass die erfindungsgemäß hergestellten Schäume beispielsweise leicht mit den oben beschriebenen Zellkulturmedien befüllt werden können. Die Zahl und Größe der Poren in den oberflächennahen Bereichen des Schaums nimmt zu. Dieses Ergebnis konnte sowohl in geschlossenen Silicongießformen als auch beim offenen Gießen (z. B. in Petrischalen oder Bechern) erreicht werden.

Die der Erfindung zugrunde liegende Aufgabe wird daher durch die in den Ansprüchen angegebene Formulierung zur Herstellung eines offenporigen Polyurethanschaums, den in den Ansprüchen angegebenen offenporigen Polyurethanschaum , das in den Ansprüchen angegebene Verfahren zur Herstellung eines derartigen Polyurethanschaums , die in den Ansprüchen angegebene Verwendung eines derartigen Polyurethanschaums , das in den Ansprüchen angegebene Verfahren zur Herstellung eines Zellträgers und den in den Ansprüchen angegebenen Zellträger gelöst.

Erfindungsgemäß wird die der vorliegenden Erfindung zugrunde liegende Aufgabe also durch eine Formulierung zur Herstellung eines offenporigen Polyurethanschaums gelöst, die eine Polyolkomponente (a), die mindestens eine Hydroxygruppen-enthaltenende Verbindung enthält, eine Polyisocyanatkomponente (b), die mindestens eine Isocyanatgruppen-enthaltende Verbindung enthält, und eine Saccharidkomponente (c), die mindestens ein Mono-, Di-, Oligo- oder Polysaccharid enthält, umfasst.

Vorzugsweise ist die Saccharidkomponente (c) in der erfindungsgemäßen Formulierung in einer Menge von 0,01 - 4,20 Gew.-%, insbesondere in einer Menge 0,5 - 3,70 Gew.-% und ganz besonders bevorzugt in einer Menge von 0,7 - 3 Gew.-% enthalten, wobei die Menge der Saccharidkomponente (c) bezogen auf die Menge der Polyolkomponente (a) (d.h. Gesamtmasse der Formulierung abzüglich der Polyisocyanatkomponente (b)) weniger als 5 Gew.-%, vorzugsweise 0,3-4,5 Gew.-% und insbesondere 0,5-4,0 Gew.-% beträgt.

Bevorzugte Saccharidkomponenten im Sinne der vorliegenden Erfindung sind dabei Monosaccharide wie Dextrose, Mannose, Mannit, Dulcit, Glucose, Fructose, Galaktose, und dergleichen, Disaccharide wie Maltose, Laktose, Saccharose, Cellobiose und dergleichen, Oligo- und Polysaccharide wie Cellulose, Pektin, Amylopektin und dergleichen, wobei Stärke ausgenommen ist, sowie Gemische von zwei oder mehr daVon, wobei Monosaccharide besonders bevorzugt sind. Speziell bevorzugte Monosaccharide sind Hexite, wie Dextrose, Mannit und Dulcit.

Saccharide als Bestandteil biokompatibler Polyurethane sind grundsätzlich bekannt. Bespielsweise beschreiben S. Wilbullucksanakul, K. Hashimoto, M. Okada, Makromol. Chem. & Phys. 1996, 197, 135-146 den Einsatz von D-Glucaro-1,4:6,3-dilacton und D-Mannaro-1,4:6,3-dilacton; U. Klügel, in DE 4430586*,* AUF Analytik Umwelttechnik, Deutschland, **1996** beschreibt den Einsatz von polysaccharidhaltiger mikrobieller Biomasse. Darin werden die Saccharide typischerweise als Monomere in hohen Anteilen eingesetzt. Ein Einfluß der Saccharide auf die Offenporigkeit der resultierenden Polyurethane wurde jedoch bislang nicht beschrieben. Diese Offenporigkeit wird erfindungsgemäß durch die Verwendung der Saccharide in relativ geringen Mengen, wie vorstehend angegeben, erreicht.

Die erfindungsgemäße Formulierung enthält als Polyolkomponente (a) eine Verbindung, die mindestens zwei Hydroxygruppen enthält, oder Gemische derartiger Verbindungen. Bevorzugte derartige Verbindungen sind dabei hydroxyterminierte Polyether wie z.B. α,ω-Dihydroxypoly(oxyethylene), α,ω-Dihydroxypoly(1,2-ethylenoxid), α,ω-Dihydroxypoly(1,2-propylenoxid), α,ω-Dihydroxypoly(1,3-trimethylenoxid), α,ω-Dihydroxypoly(1,4-tetramethylenoxid), α,ω-Dihydroxypoly(methylenoxy-1,2-ethylenoxid) und dergleichen sowie Copolymere davon, mit Molmassen vorzugsweise bis zu 15000 g/mol, hydroxyterminierte aliphatische Polycarbonate wie z.B. α,ω-Dihydroxypoly(ethylencarbonat), α,ω-Dihydroxypoly(1,2-propylencarbonat), α,ω-Dihydroxypoly(1,3-propylencarbonat), α,ω-Dihydroxypoly(tetramethylencarbonat), α,ω-Dihydroxypoly(hexamethylencarbonat) und dergleichen sowie Copolymere davon, jeweils mit Molmassen vorzugsweise bis zu 15000 g/mol, Polyanhydride aus Dicarbonsäuren, wie z.B. Malonsäure, Bernsteinsäure, Glutarsäure und dergleichen sowie Copolymere daraus, jeweils mit Molmassen vorzugsweise bis zu 15000 g/mol, niedermolekulare zwei- oder mehrwertige Alkohole wie z.B. Glykol, 1,2-Propylenglykol, 1,3-Propylenglykol, Butandiol, Pentandiol, Hexandiol und längerkettige lineare oder verzweigte aliphatische Diole, Glycerin, Triethanolamin, Pentaerythrit, 2,2-Bis(hydroxymethyl)propanol und dergleichen, Hydroxygruppen-enthaltende Aminosäuredimere, -trimere oder -oligomere, z.B. aus Tyrosin und/oder Serin, sowie Zuckeralkohole wie Sorbit und andere Naturstoffe oder Naturstoffderivate mit mindestens zwei Hydroxygruppen und dergleichen.

Bevorzugter werden jedoch als Polyolkomponente (a) Polyester eingesetzt, deren Endgruppen Hydroxygruppen sind. Beispiele derartiger Verbindungen sind Polycaprolactondiol mit einem zahlenmittleren Molekulargewicht bis zu 15000 g/mol, besonders bevorzugt von 200 g/mol bis 5000 g/mol und Polycaprolactontriol mit einem zahlenmittleren Molekulargewicht bis zu 15000 g/mol, besonders bevorzugt von 200 g/mol bis 5000 g/mol (z.B. unter der Bezeichung Capa kommerziell von Solvay erhältlich, sowie auch erhältlich im Feinchemikalienhandel). Weitere Beispiele sind α,ω-Dihydroxypoly(D,L-lactid), α,ω-Dihydroxypoly(D-lactid), α,ω-Dihydroxypoly(L-lactid), α,ω-Dihydroxypoly(glycolid), α,ω-Dihydroxypoly(hydroxybutyrat) und andere aliphatische Polyester sowie deren Copolymere einschließlich segmentierter Blockcopolymere aus Polyether- und Polyestersegmenten, wie sie z.B. aus der Umsetzung von hochmolekularen Polyestern mit hydroxyterminierten Poly(alkylenglykolen) erhalten werden können, sowie Mischungen aus derartigen Polyolen.

In der erfindungsgemäßen Formulierung ist die Polyolkomponente (a) in einer Menge von 15 - 85 Gew.-%, bevorzugter in einer Menge von 30 - 80 Gew.-% und insbesondere in einer Menge von 45-75 Gew.-% enthalten.

Gemäß der vorliegenden Erfindung wird als Polyisocyanatkomponente (b) eine Verbindung eingesetzt, die mindestens zwei Isocyanatgruppen enthält, oder Gemische derartiger Verbindungen. Eine bevorzugte derartige Verbindung ist dabei unter den Folgenden ausgewählt: gegebenenfalls substituierte Alkylendiisocyanate mit 3 bis 12 Kohlenstoffatomen wie Hexamethylendiisocyanat, oder Lysindiisocyanat, gegebenenfalls substituerte Cycloalkylendiisocyanate mit 5 bis 15 Kohlenstoffatomen wie Cyclohexylendiisocyanat, gegebenenfalls substituerte Alkylcycloalkylendiisocyanate mit 6 bis 18 Kohlenstoffatomen wie Isophorondiisocyanat, gegebenenfalls substituierte aromatische Diisocyanate wie p-Phenylendiisocyanant, Toluyldiisocyanate (alle Isomere sowie deren Mischungen), 4,4'-Diphenylmethandiisocyanat, sowie Isomere, Trimere und höhere Oligomere dieser Diisocyanate, Uretdione aus diesen Isocyanaten, Cyanurate und Isocyanurate aus diesen Isocyanaten und dergleichen. Eine besonders bevorzugt verwendete Verbindung ist Isophorondiisocyanat.

In der erfindungsgemäßen Formulierung wird die Polyisocyanatkomponente (b) in einer Menge von 8 - 70 Gew.-%, bevorzugter in einer Menge von 12 - 50 Gew.-% und insbesondere in einer Menge von 17-36 Gew.-% eingesetzt.

Darüber hinaus kann die erfindungsgemäße Formulierung zur Herstellung eines offenporigen Polyurethanschaums eine Katalysatorkomponente (d), die die Reaktion zwischen Hydroxy- und Isocyanatgruppen katalysiert und eine Treibmittelkomponente (e), die bei der Schäumtemperatur gasförmig vorliegt oder Gas bildet, umfassen.

Als Katalysatoren (d) können in der erfindungsgemäßen Formulierung basische oder Lewis-saure Verbindungen eingesetzt werden. Beispiele für basische Katalysatoren sind Diazabicycloundecen (DBU) und ähnliche cyclische oder polycyclische Amine, Morpholinderivate wie N-Alkylmorpholine, DMDEE, DMDLS und ähnliche polyfunktionelle Amine, Ethanolamine und andere dem Fachmann bekannte basische Katalysatoren zur Herstellung von Polyurethanen oder Polyharnstoffen. Beispiele für Lewis-saure Katalysatoren sind Metallkomplexe wie Dibutylzinndilaureat, Eisen-, Zirkonium- oder Vanadiumacetylacetonat, Titantetraisopropylat, und andere dem Fachmann für diesen Zweck als geeignet bekannte Lewis-saure Verbindungen. Besonders bevorzugt als Katalysator ist DBU.

In der erfindungsgemäßen Formulierung wird der Katalysator (d) im Allgemeinen in einer Menge von 0,01 - 5 Gew.-%, bevorzugt 0,1 - 1 Gew.-%, bevorzugter 0,2 - 0,7 Gew.-% eingesetzt.

Die erfindungsgemäßen Formulierungen können auch eine Treibmittelkomponente (e) für die Schaumbildung enthalten. Geeignete Treibmittel sind dabei Wasser oder organische Lösungsmittel oder Kombinationen daraus. Treibmittel und Verarbeitungstemperatur müssen so aufeinander abgestimmt sein, dass das Treibmittel bei der Verarbeitung zum Schaum durch Verdampfen (z.B. Lösungsmittel) oder durch chemische Reaktion (z.B. Wasser) Gas bildet. Bevorzugt sind daher als physikalische Treibmittel gegebenenfalls substituierte lineare, verzweigte und cyclische Alkane wie Pentan, Hexan, Heptan, Isooctan, Cyclohexan und dergleichen, Acetale wie Methylal (Dimethoxymethan) und 1,1-Dimethoxyethan, Ketone wie Aceton, Ester wie Ethylacetat, halogenierte Kohlenwasserstoffe wie Chloroform, Dichlormethan und Dichlorethan, entweder allein, in Mischungen, oder in Kombination mit Wasser als chemischem Treibmittel. Besonders bevorzugt sind Acetale wie Methylal (Dimethoxymethan) und 1,1-Dimethoxyethan. Als Treibmittel können auch Feststoffe dienen, die bei der Reaktionstemperatur, d.h. bei Temperaturen unter 100°C, Gase freisetzen. Als Beispiele können anorganische Verbindungen wie Ammoniumsalze, vorzugsweise Ammoniumcarbonat, Ammoniumhydrogencarbonat und Ammoniumoxalat, oder auch organische Verbindungen, wie Carbazide, Hydrazide (z.B. Benzolsulfohydrazid), Azoverbindungen und Diazoverbindungen, genannt werden. Die Feststoffe oder deren Lösungen können allein oder im Gemisch mit mindestens einem physikalischen und/oder chemischen Treibmittel eingesetzt werden.

Entsprechend der Erfindung werden die physikalischen Treibmittel in Mengen von 7 - 30 Gew.-%, die festen Treibmittel in Mengen von 1-5 Gew.-% und Wasser in Mengen von 0,01 - 1 Gew.-% eingesetzt.

Des Weiteren kann die erfindungsgemäße Formulierung ein oder mehrere zusätzliche, dem Fachmann geläufige Additive enthalten. Derartige Additive sind beispielsweise Verdünnungsmittel, Weichmacher, Tenside, Schaumstabilisatoren, Nukleierungsmittel, Verbindungen zur Einstellung der Oberflächenspannung und Polarität, Viskositätsverbesserer und dergleichen. Beispiele für Zusätze, die solche Funktionen in diesen Formulierungen erfüllen können, sind amphiphile Polymere (z.B. Pluronics, PEO/PPO Copolymere oder Blockcopolymere, teilverseiftes Poly(vinylacetat)), Siliconöle, anorganische Partikel, z.B. aus Tricalciumphosphat, Hydroxyapatit und ähnlichem, Kochsalz und andere Salze und Aminosäuren und dergleichen.

Zur Bildung eines Polyurethanschaums wird die erfindungsgemäße Formulierung bei einer Temperatur von 20°C - 70 °C vorgemischt, wobei vorzugsweise zunächst Polyolkomponente (a) und Saccharidkomponente (c) vermischt werden und dann Polyisocyanatkomponente (b) zugegeben wird. Anschließend wird die Formulierung in eine entsprechende Form eingefüllt und auf eine Temperatur erwärmt, die zur Herbeiführung der Polymerisationsreaktion ausreichend ist. Üblicherweise liegt diese Temperatur im Bereich von 30°C bis 90°C. Nachfolgend wird der Polyurethanschaum bis zu seinem vollständigen Aushärten bei dieser Temperatur gehalten, was üblicherweise nach etwa 1 min bis 24h der Fall ist. Danach kann die Form entfernt werden. Abschließend kann die Hydrophilie des Polyurethanschaums durch zusätzliche Behandlung in Alkoholen, Wasser oder wässrigen Lösungen wie z.B. Kulturmedien für Zellkulturen, gegebenenfalls in schrittweisem Übergang, bei Raumtemperatur oder erhöhten Temperaturen verbessert werden. Üblicherweise wird diese Behandlung in einer oder mehreren Stufen über einen Zeitraum von etwa 1 min bis 24 h durchgeführt.

Zusätzlich können die Porenstruktur und die Interkonnektivität der Poren des Polyurethanschaums durch Anlegen eines Unterdrucks am Ende des Schäumvorgangs verbessert werden.

Die vorliegende Erfindung beschreibt eine allgemeine Lösungsmöglichkeit zur Verhinderung der Hautbildung, die insbesondere für die Herstellung biokompatibler und bioabbaubarer Polyurethanschäume für medizinische Anwendungen wie Tissue Engineering geeignet sind.

Da die erfindungsgemäßen Polyurethanschäume nicht toxisch und gut biokompatibel sind, mit gängigen Verfahren sterilisiert werden können sowie eine gute Hydrophilie aufweisen, was für die Anlagerung von Zellen auf der Oberfläche von großer Bedeutung ist, stellen sie besonders auch im Bereich der Schäume für das Tissue Engineering einen bedeutenden Fortschritt dar.

Ein Verfahren des Tissue Engineering, für das die erfindungsgemäß hergestellten Schäume in besonderem Maße geeignet sind, besteht darin, dass die Form der gewünschten Implantate mittels bildgebender Verfahren (z.B. Ultraschall, Computertomographie etc.) am Patienten aufgenommen werden kann. Auf Basis dieser Bilddaten wird durch Laserstereolithographie ein Modell erstellt und davon eine Negativform aus einem geeigneten Material wie Silicon hergestellt. In dieser Negativform kann durch ein einfaches Gießverfahren das Scaffold (der Zellträger) als offenporiger Schaum ohne Haut aus physiologisch abbaubarem und biokompatiblem Polyurethan gebildet werden. Dieser Zellträger hat dann genau die Form, die der Stelle entspricht, an die bei diesem Patienten das Implantat gesetzt werden soll. Der Zellträger wird mit Zellen (direkt aus Suspension oder eingebettet in einem Gel (z. B. Fibrinkleber), das auch zellbiologische Botenstoffe wie Wachstumsfaktoren etc. enthalten kann) gefüllt. Dieser Füllvorgang ist nur möglich, wenn man einen vollständig offenporigen Schaum hat, der keine Haut aufweist.

Implantate, die durch dieses Verfahren hergestellt werden, können insbesondere als Ersatz für Knorpel, z.B. im Bereich der Ohren, der Nase, der Bandscheiben, des Meniskus sowie in Situationen, in denen Knorpel-Knochen-Verbindungen notwendig sind wie z.B. bei Gelenkknorpeln (z.B. Knie) und dergleichen eingesetzt werden.

Im Folgenden wird die vorliegende Erfindung unter Bezugnahme auf die beigefügten Zeichnungen weiter erläutert. Die aufgeführten Beispiele dienen dabei zur Veranschaulichung der Erfindung und sollen nicht als einschränkend aufgefasst werden.

Figur 1 zeigt eine Rasterelektronenmikroskop-(REM)-Photographie eines Polyurethanschaums des Standes der Technik, der in Vergleichsbeispiel 1 erhalten worden ist, Figur 2 zeigt dagegen eine REM-Photographie des in Beispiel 1 aus einer erfindungsgemäßen Formulierung erhaltenen Polyurethanschaums.

In den Figuren bedeuten 1 die Oberfläche des jeweiligen Polyurethanschaums und 2 einen Anschnitt in das Innere des entsprechenden Polyurethanschaums.

### Beispiele

Bekanntermaßen kann die Offenporigkeit von Polyurethanschäumen sowohl durch Additive als auch durch verarbeitungstechnische Operationen beeinflusst werden. Die folgenden Beispiele (außer Vergleichsbeispiel 1) stehen alle für offenporige Schäume und zeigen, wie erfindungsgemäß auch die Offenporigkeit an der Formteiloberfläche bzw. an den Wandungen einer Siliconform erreicht werden kann. Alle Prozentangaben in den Beispielen beziehen sich auf das Gewicht.

### Vergleichsbeispiel 1

Eine Formulierung aus 24% Polycaprolactondiol (Mₙ = 1250), 20% Polycaprolactontriol (Mₙ = 900), 36% Isophorondiisocyanat, 1,6% Polyethylenglycol-*block-*Polypropylenglycol-*block*-Polyethylenglycol (Mₙ = 14600), 4 % Triethanolamin, 0,08% Wasser, 2,5% Celluloseacetatbutyrat, 0,25% Diazabicycloundecen und 11,57 % Cyclohexan wird nach dem gründlichen Vermischen bei 60 °C in einer Petrischale oder in einer Siliconform für vier Stunden auf 75°C erwärmt. Wie aus Figur 1 deutlich ersichtlich ist, ist die erhaltene Formulierung zwar offenporig, jedoch sind die meisten Poren zur Oberfläche hin durch eine Haut abgeschlossen.

### Beispiel 1

Eine Formulierung aus 24% Polycaprolactondiol (Mn = 1250), 20% Polycaprolactontriol (Mn = 900), 36% Isophorondiisocyanat, 1,6% Polyethylenglycol-block-Polypropylenglycol-block-Polyethylenglycol (Mn = 14600), 4% Triethanolamin, 0,08% Wasser, 2,5% Dextrose (entspricht 3.9% bezogen auf die Polyolkomponente, wobei die Polyolkomponente alle Komponenten außer dem Diisocyanat enthält), 0,25% Diazabicycloundecen und 11,57% Cyclohexan wird nach dem gründlichen Vermischen bei 60 °C in einer Petrischale für vier Stunden auf 75°C erwärmt. Das entstehende Formteil ist gleichmäßig porös. Figur 2 zeigt deutlich, dass die Interkonnektivität der Poren gut ausgebildet ist. Im Gegensatz zum Polyurethanschaum aus Vergleichsbeispiel 1 sind in diesem Fall auch die Poren an der Oberfläche offen. Der Schaum wird nach der Herstellung eine Stunde in Wasser gekocht, wodurch die Hydrophilie verbessert wird. Danach kann er z. B. leicht mit Fibrinkleber, der eine Zellkultur (z. B. Chondrocyten, Fibroblasten oder Osteoblasten) enthält, beladen werden.

### Beispiel 2

Eine Formulierung wie in Beispiel 1 beschrieben wird in eine Siliconform mit nur einem Anguss, die mittels eines Stereolithographiemodells nach einem menschlichen Ohr gefertigt wurde, gegossen. Die Form wurde zuvor auf 70°C vorgeheizt und die Temperatur wird zum Aushärten auch noch 8 Stunden beibehalten. Man erhält ein gleichmäßig geschäumtes Formteil. Die Poren an der Oberfläche sind geöffnet und interkonnektieren mit den Poren im Inneren. Auch bei diesem Formteil wird durch Kochen in Wasser oder physiologischer Kochsalzlösung die Hydrophilie verbessert. Das erhaltene Formteil kann zur Herstellung eines Implantats mit einer Mischung aus Zellen, Fibrinkleber und verschiedenen Wachstumsfaktoren (Growth Factors) leicht befüllt werden.

### Beispiel 3

Beispiel 3 zeigt die Eignung des biokompatiblen Nukleierungsmittels Tricalciumphosphat in Form von Nanopartikeln in der erfindungsgemäßen Formulierung.

In Polycaprolactondiol (Mn = 2000) werden 40% Tricalciumphosphatnanopartikel als Nukleierungsmittel dispergiert. Es wird eine Formulierung hergestellt, die 14,0% dieser Dispersion enthält sowie weiterhin 21,3% Polycaprolactondiol (Mn = 2000), 21,3% Polycaprolactontriol (Mn = 900), 17,7% Isophorondiisocyanat, 1,8% Polyethylenglycol-block-Polypropylenglycol-block-Polyethylenglycol (Mn = 14600), 2,1% Triethanolamin, 0,07% Wasser, 2,1% Dextrose (entspricht 2.55% bezogen auf die Polyolkomponente, wobei die Polyolkomponente alle Komponenten außer dem Diisocyanat enthält), 1,4% Octanol, 0,21% Diaminobicycloundecen und 18,02% Cyclohexan. Diese Formulierung wird nach dem gründlichen Vermischen bei 55°C in einer Petrischale für vier Stunden auf 75°C erwärmt. Das entstehende Formteil ist gleichmäßig porös. Die Interkonnektivität der Poren ist gut, die Poren an der Oberfläche sind offen. Der Schaum wird nach der Herstellung eine Stunde in Wasser gekocht, wodurch sich die Hydrophilie verbessert. Dann kann er z. B. leicht mit Fibrinkleber, der eine Zellkultur (z. B. Chondrocyten, Fibroblasten oder Osteoblasten) enthält, beladen werden.

### Beispiel 4

Das Anlegen eines Unterdrucks am Ende des Schäumvorgangs kann die Porenstruktur und Interkonnektivität der Poren zusätzlich verbessern.

Eine Formulierung aus 51% Polycaprolactontriol (Mn = 900), 21,4% Isophorondiisocyanat, 0,25% Schaumstabilisator DABCO 3042, 1,05% Dextrose (entspricht 1.33% bezogen auf die Polyolkomponente, wobei die Polyolkomponente alle Komponenten außer dem Diisocyanat enthält), 0,5% Diazabicycloundecen und 25,8% Hexan wird nach dem gründlichen Vermischen bei 55°C in einer Petrischale für vier Stunden auf 67°C erwärmt. Das entstehende Formteil ist gleichmäßig porös. Der Schaum wird noch 48 Stunden in einem Exsikkator auf 500 mbar evakuiert. Zur Verbesserung der Hydrophilie wird er 2 min mit Ethanol und anschließend mit Modified Eagle's Medium (Zellkulturmedium) behandelt. Dann kann er leicht mit Fibrinkleber, der eine Zellkultur (z. B. Chondrocyten, Fibroblasten oder Osteoblasten) enthält, beladen werden.

### Beispiel 5

Eine Formulierung aus 25,1% Polycaprolactontriol (Mn = 900), 50,2% Polycaprolactondiol (CAPA 2402, Mn = 4000), 15,25% Isophorondiisocyanat, 0,25% Schaumstabilisator DABCO 3042, 1,05% Dextrose (entspricht 1.24% bezogen auf die Polyolkomponente, wobei die Polyolkomponente alle Komponenten außer dem Diisocyanat enthält), 0,5% Diazabicycloundecen und 7,65% Hexan wird nach dem gründlichen Vermischen bei 58°C in einer Petrischale für vier Stunden auf 67°C erwärmt. Das entstehende Formteil ist gleichmäßig porös. Der Schaum wird in einem Exsikkator 48 Stunden lang auf 500 mbar evakuiert. Zur Verbesserung der Hydrophilie wird er 2 min mit Ethanol und anschließend mit Modified Eagle's Medium behandelt. Dann kann er z. B. leicht mit Fibrinkleber, der eine Zellkultur (z. B. Chondrocyten, Fibroblasten oder Osteoblasten) enthält, beladen werden.

### Beispiel 6

Eine Formulierung aus 33,1% Polycaprolactontriol (Mn = 900), 33,1% Polycaprolactondiol (Mn = 2000), 4,15% Polyethylenglykol (PEG; Mn = 600), 18,75% Isophorondiisocyanat, 0,3% Schaumstabilisator DABCO 3042,1,3% Dextrose (entspricht 1.6% bezogen auf die Polyolkomponente, wobei die Polyolkomponente alle Komponenten außer dem Diisocyanat enthält), 0,65% Diazabicycloundecen und 8,65% Hexan wird nach dem gründlichen Vermischen bei 58°C in einer Petrischale für vier Stunden auf 67°C erwärmt. Das entstehende Formteil ist gleichmäßig porös. Der Schaum wird in einem Exsikkator 48 Stunden lang auf 500 mbar evakuiert. Zur Verbesserung der Hydrophilie wird er 2 min mit Ethanol und anschließend mit Modified Eagle's Medium behandelt. Dann kann er z. B. leicht mit Fibrinkleber, der eine Zellkultur (z. B. Chondrocyten, Fibroblasten oder Osteoblasten) enthält, beladen werden.

### Beispiel 7

Eine Formulierung aus 31,19% Polycaprolactontriol (Mn = 900), 31,19% Polycaprolactondiol (Mn = 2000), 16,81% Isophorondiisocyanat, 1,14% Dextrose (entspricht 1.37% bezogen auf die Polyolkomponente, wobei die Polyolkomponente alle Komponenten außer dem Diisocyanat enthält), 0,31% Schaumstabilisator DABCO 3042, 18,71% Methylal und 0,65% Diazabicycloundecen wird nach gründlichem Vermischen in einer Petrischale bei 30°C mit einer Spritze in eine auf 67°C vorgewärmte Siliconform (in der Form des knorpeligen Anteils einer menschlichen Ohrmuschel) eingefüllt und drei Stunden auf 67°C gehalten. Das entstehende Formteil ist gleichmäßig porös, die Poren sind miteinander verbunden und die an der Oberfläche liegenden Poren sind offen. Es wird keine erkennbare Haut beim Schäumen gebildet. Das Formteil wird in einem Exsikkator 24 Stunden auf 3 mbar evakuiert. Zur Verbesserung der Hydrophilie wird es 5 min mit Ethanol behandelt und anschließend mehrfach in Dulbeco's Modified Eagle's Medium gewaschen. Es wird mit humanen Chondrocyten besiedelt und in vierwöchiger Kultur bildet sich ein zusammenhängendes knorpelartiges Gewebe.

### Beispiel 8

Eine Formulierung aus 33,7% Polycaprolactontriol (Mn = 900), 33,7% Polycaprolactondiol (Mn = 2000), 18,0% Isophorondiisocyanat, 0,7% Mannit (entspricht 0,85% bezogen auf die Polyolkomponente, wobei die Polyolkomponente alle Komponenten außer dem Diisocyanat enthält), 0,3% Schaumstabilisator DABCO 3042, 13,2% Methylal und 0,4% Diazabicycloundecen wird nach gründlichem Vermischen in einer Petrischale bei 30°C mit einer Spritze in eine auf 67°C vorgewärmte Siliconform (in der Form des knorpeligen Anteils einer menschlichen Ohrmuschel) eingefüllt und drei Stunden auf 67°C gehalten. Das entstehende Formteil ist gleichmäßig porös, die Poren sind miteinander verbunden und die an der Oberfläche liegenden Poren sind offen. Es wird keine erkennbare Haut beim Schäumen gebildet. Das Formteil wird in einem. Exsikkator 24 Stunden auf 3 mbar evakuiert. Zur Verbesserung der Hydrophilie wird es 5 min mit Ethanol behandelt und anschließend mehrfach in Dulbeco's Modified Eagle's Medium gewaschen. Die Formulierung ist auch bei 134°C mit Wasserdampf sterilisierbar, ohne Veränderungen zu zeigen.

## Patentansprüche

1. Formulierung zur Herstellung eines offenporigen Polyurethanschaums, der keine Haut zur Außenseite aufweist, umfassend
(a) eine Polyolkomponente, die mindestens eine Hydroxygruppenenthaltenende Verbindung enthält, in einer Menge von 15 bis 85 Gew.-%,
(b) eine Polyisocyanatkomponente, die mindestens eine Isocyanatgruppen-enthaltende Verbindung enthält, in einer Menge von 8 bis 70 Gew.-%, und
(c) eine Saccharidkomponente, die mindestens ein Mono-, Di-, Oligo- oder Polysaccharid, ausgenommen Stärke, enthält, in einer Menge von 0,01 - 4,20 Gew.-%, wobei die Menge der Saccharidkomponente bezogen auf die Polyolkomponente (a) weniger als 5 Gew.-% beträgt.

2. Formulierung zur Herstellung eines offenporigen Polyurethanschaums nach Anspruch 1, wobei die Saccharidkomponente (c) mindestens ein Mono, Di-, Oligo- oder Polysaccharid enthält, ausgewählt aus der Gruppe, umfassend Dextrose, Mannose, Mannit, Dulcit, Glucose, Fructose, Galaktose, Maltose, Laktose, Saccharose, Cellobiose, Cellulose, Pektin, Amylopektin und Gemische von zwei oder mehreren davon.

3. Formulierung zur Herstellung eines offenporigen Polyurethanschaums nach einem der vorangehenden Ansprüche, wobei die Saccharidkomponente (c) in einer Menge von 0,5 - 3,70 Gew.-% enthalten ist.

4. Formulierung zur Herstellung eines offenporigen Polyurethanschaums nach einem der vorangehenden Ansprüche, wobei die Saccharidkomponente (c) in einer Menge von 0,7 - 3 Gew.-% enthalten ist.

5. Formulierung zur Herstellung eines offenporigen Polyurethanschaums nach einem der vorangehenden Ansprüche, wobei die Polyolkomponente (a) mindestens eine Hydroxygruppen-enthaltende Verbindung enthält, ausgewählt aus der Gruppe, umfassend hydroxyterminierte Polyether, Copolymere hydroxyterminierter Polyether, hydroxyterminierte aliphatische Polycarbonate, Copolymere hydroxyterminierter aliphatischer Polycarbonate, Polyanhydride von Dicarbonsäuren, Copolymere dieser Polyanhydride, niedermolekulare zwei- oder mehrwertige Alkohole, Hydroxygruppen-enthaltende Aminosäuredimere, -trimere oder -oligomere, Zuckeralkohole, andere Naturstoffe oder Naturstoffderivate mit mindestens zwei Hydroxygruppen, Polyester, deren Endgruppen Hydroxygruppen sind, sowie Copolymere derartiger Polyester, segmentierte Blockcopolymere aus Polyether- und Polyestersegmenten und Gemische von zwei oder mehreren davon.

6. Formulierung zur Herstellung eines offenporigen Polyurethanschaums nach Anspruch 5, wobei die Polyolkomponente (a) mindestens eine Hydroxygruppen-enthaltende Verbindung enthält, ausgewählt aus der Gruppe, umfassend α,ω-Dihydroxypoly(oxyethylen), α,ω-Dihydroxypoly(1,2-ethylenoxid), α,ω-Dihydroxypoly(1,2-propylenoxid), α,ω-Dihydroxypoly(1,3-trimethylenoxid), α,ω-Dihydroxypoly(1,4-tetramethylenoxid), α,ω-Dihydroxypoly(methylenoxy-1,2-ethylenoxid) sowie Copolymere davon, jeweils mit Molmassen bis zu 15000 g/mol, α,ω-Dihydroxypoly(ethylencarbonat), α,ω-Dihydroxypoly(1,2-propylencarbonat), α,ω-Dihydroxypoly(1,3-propylencarbonat), α,ω-Dihydroxypoly(tetramethylencarbonat), α,ω-Dihydroxypoly(hexamethylencarbonat), Copolymere davon, jeweils mit Molmassen bis zu 15000 g/mol, Polyanhydride aus Malonsäure, Bernsteinsäure, Glutarsäure oder Gemischen davon, sowie Copolymere daraus, jeweils mit Molmassen bis zu 15000 g/mol, Polycaprolactondiol mit einem zahlenmittleren Molekulargewicht bis zu 15000 g/mol, Polycaprolactontriol mit einem zahlenmittleren Molekulargewicht bis zu 15000 g/mol, Glykol, 1,2-Propylenglykol, 1,3-Propylenglykol, Butandiol, Pentandiol, Hexandiol, Glycerin, Triethanolamin, Pentaerythrit, 2,2-Bis(hydroxymethyl)propanol, Hydroxygruppen-enthaltende Aminosäuredimere, -trimere oder -oligomere aus Tyrosin und/oder Serin, sowie Sorbit und Gemische von zwei oder mehreren davon.

7. Formulierung zur Herstellung eines offenporigen Polyurethanschaums nach Anspruch 5, wobei die Polyolkomponente (a) mindestens eine Hydroxygruppen-enthaltende Verbindung enthält, ausgewählt aus der Gruppe, umfassend Polycaprolactondiol mit einem zahlenmittleren Molekulargewicht von 200 g/mol bis 5000 g/mol, Polycaprolactontriol mit einem zahlenmittleren Molekulargewicht von 200 g/mol bis 5000 g/mol und Gemische davon.

8. Formulierung zur Herstellung eines offenporigen Polyurethanschaums nach einem der vorangehenden Ansprüche, wobei die Polyolkomponente (a) in einer Menge von 30 bis 80 Gew.-% enthalten ist.

9. Formulierung zur Herstellung eines offenporigen Polyurethanschaums nach Anspruch 8, wobei die Polyolkomponente (a) in einer Menge von 45 bis 75 Gew.-% enthalten ist.

10. Formulierung zur Herstellung eines offenporigen Polyurethanschaums nach einem der vorangehenden Ansprüche, wobei die Polyisocyanatkomponente (b) mindestens eine Isocyanatgruppen-enthaltende Verbindung enthält, ausgewählt aus der Gruppe, umfassend
gegebenenfalls substituierte Alkylendiisocyanate mit 3 bis 12 Kohlenstoffatomen, gegebenenfalls substituerte Cycloalkylendiisocyanate mit 5 bis 15 Kohlenstoffatomen, gegebenenfalls substituerte Alkylcycloalkylendiisocyanate mit 6 bis 18 Kohlenstoffatomen, gegebenenfalls substituierte aromatische Diisocyanate, Isomere, Trimere und höhere Oligomere dieser Diisocyanate, Uretdione aus diesen Isocyanaten, Cyanurate und Isocyanurate aus diesen Isocyanaten und Gemische von zwei oder mehreren davon.

11. Formulierung zur Herstellung eines offenporigen Polyurethanschaums nach Anspruch 10, wobei die Polyisocyanatkomponente (b) mindestens eine Isocyanatgruppen-enthaltende Verbindung enthält, ausgewählt aus der Gruppe, umfassend
Hexamethylendiisocyanat, Tetramethylendiisocyanat, Lysindiisocyanat, Cyclohexylendiisocyanat, Isophorondiisocyanat, p-Phenylendüsocyanat, Toluyldiisocyanate, Isomere, Trimere und höhere Oligomere dieser Diisocyanate, Uretdione aus diesen Isocyanaten, Cyanurate und Isocyanurate aus diesen Isocyanaten und Gemische von zwei oder mehreren davon.

12. Formulierung zur Herstellung eines offenporigen Polyurethanschaums nach einem der vorangehenden Ansprüche, wobei die Polyisocyanatkomponente (b) in einer Menge von 12 bis 50 Gew.-% enthalten ist.

13. Formulierung zur Herstellung eines offenporigen Polyurethanschaums nach Anspruch 12, wobei die Polyisocyanatkomponente (b) in einer Menge von 17 bis 36 Gew.-% enthalten ist.

14. Formulierung zur Herstellung eines offenporigen Polyurethanschaums nach einem der vorangehenden Ansprüche, wobei die Formulierung zusätzlich
(d) eine Katalysatorkomponente, die eine basische oder Lewis-saure Verbindung enthält, und/oder
(e) eine Treibmittelkomponente, die ein organisches Lösungsmittel als physikalisches Treibmittel, einen Feststoff oder Wasser als chemisches Treibmittel oder eine Kombination daraus enthält, umfasst.

15. Formulierung zur Herstellung eines offenporigen Polyurethanschaums nach Anspruch 14, wobei die Katalysatorkomponente (d) in einer Menge von 0,01 bis 5 Gew.-% enthalten ist.

16. Formulierung zur Herstellung eines offenporigen Polyurethanschaums nach Anspruch 15, wobei die Katalysatorkomponente (d) in einer Menge von 0,1 bis 1 Gew.-% enthalten ist.

17. Formulierung zur Herstellung eines offenporigen Polyurethanschaums nach einem der Ansprüche 14 bis 16, wobei das organische Lösungsmittel der Treibmittelkomponente (e) ausgewählt ist aus der Gruppe umfassend gegebenenfalls substituierte lineare, verzweigte und cyclische Alkane, Acetale, Ketone, Ester, halogenierte Kohlenwasserstoffe oder Gemische davon.

18. Formulierung zur Herstellung eines offenporigen Polyurethanschaums nach Anspruch 17, wobei das organische Lösungsmittel der Treibmittelkomponente (e) ausgewählt ist aus der Gruppe umfassend Pentan, Hexan, Heptan, Isooctan, Cyclohexan, Dimethoxymethan, 1,1-Dimethoxyethan, Aceton, Ethylacetat, Chloroform, Dichlormethan und Dichlorethan.

19. Formulierung zur Herstellung eines offenporigen Polyurethanschaums nach einem der Ansprüche 14 bis 18, wobei der Feststoff der Treibmittelkomponente (e) eine anorganische oder organische Verbindung ist, ausgewählt aus der Gruppe, umfassend Ammoniumcarbonat, Ammoniumhydrogencarbonat, Ammoniumoxalat, Carbazide, Hydrazide, Azoverbindungen und Diazoverbindungen.

20. Formulierung zur Herstellung eines offenporigen Polyurethanschaums nach einem der Ansprüche 14 bis 19, wobei das organische Lösungsmittel der Treibmittelkomponente (e) in einer Menge von 7 bis 30 Gew.-%, das feste Treibmittel in einer Menge von 1 bis 5 Gew.-% und das Wasser der Treibmittelkomponente in einer Menge von 0,01 bis 1 Gew.-% enthalten ist.

21. Formulierung zur Herstellung eines offenporigen Polyurethanschaums nach einem der vorangehenden Ansprüche, wobei die Formulierung gegebenenfalls einen oder mehrere weitere Inhaltsstoffe enthält, ausgewählt aus der Gruppe umfassend Verdünnungsmittel, Weichmacher, Tenside, Schaumstabilisatoren, Nukleierungsmittel, Verbindungen zur Einstellung der Oberflächenspannung und Polarität, Viskositätsverbesserer, Siliconöle, anorganische Partikel oder Nanopartikel, Kochsalz und andere Salze sowie Aminosäuren.

22. Offenporiger Polyurethanschaum, erhältlich durch Erwärmen einer Formulierung nach einem der Ansprüche 1 bis 21 auf eine Temperatur von etwa 30°C bis etwa 90 °C über einen Zeitraum von etwa 1 min bis etwa 24 h.

23. Offenporiger Polyurethanschaum nach Anspruch 22, wobei der Polyurethanschaum biokompatibel und/oder bioabbaubar ist.

24. Offenporiger Polyurethanschaum nach Anspruch 22 oder 23, wobei die Hydrophilie des Polyurethanschaums durch Kochen in Wasser über einen Zeitraum von etwa 1 min bis etwa 24 h weiter erhöht worden ist und/oder durch Behandlung mit Alkohol, Wasser oder Kulturmedium in schrittweisem Übergang eingestellt worden ist.

25. Verfahren zur Herstellung eines offenporigen Polyurethanschaums nach einem der Ansprüche 22 bis 24, wobei die Komponenten der Formulierung nach einem der Ansprüche 1 bis 21 miteinander vermischt werden und das Gemisch über einen Zeitraum von etwa 3 min bis etwa 24 h auf eine Temperatur von etwa 30 °C bis etwa 90 °C erwärmt wird.

26. Verfahren nach Anspruch 25, wobei der Polyurethanschaum des Weiteren über einen Zeitraum von 1 min bis 24 h in Wasser gekocht wird.

27. Verfahren nach Anspruch 25 oder Anspruch 26, wobei der Polyurethanschaum des Weiteren in schrittweisem Übergang mit Alkohol, Wasser oder Kulturmedium behandelt worden ist.

28. Verwendung eines offenporigen Polyurethanschaums nach einem der Ansprüche 22 bis 24 als Filtermedium oder Schwamm.

29. Verwendung eines offenporigen Polyurethanschaums nach einem der Ansprüche 22 bis 24 in der Medizin.

30. Verwendung eines offenporigen Polyurethanschaums nach einem der Ansprüche 22 bis 24 als Trägermaterial für Arzneimittel.

31. Verwendung eines offenporigen Polyurethanschaums nach einem der Ansprüche 22 bis 24 als Träger für Zellkulturen.

32. Verwendung eines offenporigen Polyurethanschaums nach einem der Ansprüche 22 bis 24 als Träger für Gewebekulturen.

33. Verwendung eines offenporigen Polyurethanschaums nach einem der Ansprüche 22 bis 24 zur Herstellung eines Zellträgers für das Tissue Engineering.

34. Verwendung eines offenporigen Polyurethanschaums nach Anspruch 33, wobei der Zellträger zum Aufbau von Implantaten als Ersatz für Knorpel und in Situationen, in denen Knorpel-Knochen-Verbindungen notwendig sind, dient.

35. Verwendung eines offenporigen Polyurethanschaums nach Anspruch 34, wobei die Implantate als Ersatz für Knorpel im Bereich der Ohren, der Nase, der Bandscheiben oder des Meniskus dienen.

36. Verfahren zur Herstellung eines Zellträgers für das Tissue Engineering aus einem offenporigen Polyurethanschaum nach einem der Ansprüche 22 bis 24, das die folgenden Stufen umfasst:
(a) Aufnahme der äußeren Form eines gewünschten Implantats durch ein bildgebendes Verfahren an einem Patienten,
(b) Erstellen eines Negativmodells dieses Implantats als Gussform aus einem geeigneten Material unter Verwendung der in Stufe (a) erhaltenen Daten,
(c) Ausgießen des Negativmodells mit einer Formulierung zur Herstellung eines offenporigen Polyurethanschaums nach einem der Ansprüche 1 bis 21,
(d) Aushärten der Formulierung und
(e) Entfernen des Negativmodells unter Erhalt des Zellträgers aus einem offenporigen Polyurethanschaum.
(f) Konditionierung für die Besiedlung mit Zellen oder zellhaltigen Trägermedien durch Kochen in Wasser oder durch die Behandlung mit Alkohol, Wasser oder Kulturmedium in schrittweisem Übergang.

37. Verfahren nach Anspruch 36, wobei das Material der Gussform in Stufe (b) ein Silicon ist.

38. Zellträger, erhalten durch das Verfahren nach einem der Ansprüche 36 und 37.

## Claims

1. Formulation for the preparation of an open-cell polyurethane foam having no skin at the exterior comprising
(a) a polyol component containing at least one hydroxyl group containing compound in an amount of 15 to 85% by weight.
(b) a polyisocyanate component containing at least one isocyanate group containing compound in an amount of 8 to 70% by weight and
(c) a saccharide component containing at least one monosaccharide, disaccharide, oligosaccharide or polysaccharide provided that starch is excluded, in an amount of 0.01 to 4.20% by weight wherein the amount of the saccharide component based on the polyol component (a), amounts to less than 5% by weight.

2. Formulation for the preparation of an open-cell polyurethane foam according to claim 1, wherein the saccharide component (c) includes at least one monosaccharide, disaccharide, oligosaccharide or polysaccharide selected from the group comprising dextrose, mannose, mannitol, dulcitol, glucose, fructose, galactose, maltose, lactose, saccharose, cellobiose, cellulose, pectin, amylopectin and mixtures of two or more thereof.

3. Formulation for the preparation of an open-cell polyurethane foam according to anyone of the preceding claims, wherein the saccharide component (c) is contained in an amount of 0.5 to 3.70% by weight.

4. Formulation for the preparation of an open-cell polyurethane foam according to anyone of the preceding claims, wherein the saccharide component (c) is contained in an amount of 0.7 to 3% by weight.

5. Formulation for the preparation of an open-cell polyurethane foam according to anyone of the preceding claims, wherein the polyol component (a) contains at least one hydroxyl group containing compound selected from the group comprising
hydroxyl group terminated polyethers, copolymers of hydroxyl group terminated polyethers, hydroxyl group terminated aliphatic polycarbonates, copolymers of hydroxyl group terminated aliphatic polycarbonates, polyanhydrides of dicarboxylic acids, copolymers of these polyanhydrides, low molecular weight bivalent or polyvalent alcohols, hydroxyl group containing amino acid dimers, amino acid trimers or amino acid oligomers, sugar alcohols, other natural products or derivatives of natural products having at least two hydroxyl groups, polyesters having hydroxyl groups as terminals as well as copolymers of such polyesters, segmented block copolymers of polyether segments and polyester segments and mixtures of two or more thereof.

6. Formulation for the preparation of an open-cell polyurethane foam according to claim 5, wherein the polyol component (a) contains at least one hydroxyl group containing compound selected from the group comprising
α,ω-dihydroxy poly(oxyethylene), α,ω-dihydroxy poly(1,2-ethylene oxide), α,ω-dihydroxy poly(1,2-propylene oxide), α,ω-dihydroxy poly(1,3-trimethylene oxide), α,ω-dihydroxy poly(1,4-tetramethylene oxide), α,ω-dihydroxy poly(methyleneoxy-1,2-ethylene oxide) as well as copolymers thereof, each of which has a molecular weight of up to 15,000 g/mol, α,ω-dihydroxy poly(ethylene carbonate), α,ω-dihydroxy poly(1,2-propylene carbonate), α,ω-dihydroxy poly(1,3-propylene carbonate), α,ω-dihydroxy poly(tetramethylene carbonate), α,ω-dihydroxy poly(hexamethylene carbonate), copolymers thereof, each of which has a molecular weight of up to 15,000 g/mol, polyanhydrides of malonic acid, succinic acid, glutaric acid or mixtures thereof as well as copolymers thereof, each of which has a molecular weight of up to 15,000 g/mol, polycaprolactone triol having a number average molecular weight of up to 15,000 g/mol, polycaprolactone diol having a number average molecular weight of up to 15000 g/mol, glycol, 1,2-propylene glycol, 1,3-proplylene glycol, butanediol, pentanediol, hexanediol, glycerine, triethanolamine, pentaerythritol, 2,2-bis(hydroxymethyl)propanol, hydroxyl group containing amino acid dimers , amino acid trimers and amino acid oligomers of tyrosine and/or serine, as well as sorbitol and mixtures of two or more thereof.

7. Formulation for the preparation of an open-cell polyurethane foam according to claim 5, wherein the polyol component (a) contains at least one hydroxyl group containing compound selected from the group comprising polycaprolactone diol having a number average molecular weight of 200 g/mol to 5,000 g/mol, polycaprolactone triol having a number average molecular weight of 200 g/mol to 5,000 g/mol and mixtures thereof.

8. Formulation for the preparation of an open-cell polyurethane foam according to anyone of the preceding claims, wherein the polyol component (a) is contained in an amount of 30 to 80% by weight.

9. Formulation for the preparation of an open-cell polyurethane foam according to claim 8, wherein the polyol component (a) is contained in an amount of 45 to 75% by weight.

10. Formulation for the preparation of an open-cell polyurethane foam according to anyone of the preceding claims, wherein the polyisocyanate component (b) contains at least one isocyanate group containing compound selected from the group comprising
optionally substituted alkylene diisocyanates having 3 to 12 carbon atoms, optionally substituted cycloalkylene diisocyanates having 5 to 15 carbon atoms, optionally substituted alkylcycloalkylene diisocyanates having 6 to 18 carbon atoms, optionally substituted aromatic diisocyanates, isomers, trimers and higher oligomers of these diisocyanates, uretdiones of these isocyanates, cyanurates and isocyanurates of these isocyanates and mixtures of two or more thereof.

11. Formulation for the preparation of an open-cell polyurethane foam according to claim 10, wherein the polyisocyanate component (b) contains at least one isocyanate group containing compound selected from the group comprising
hexamethylene diisocyanate, tetramethylene diisocyanate, lysine diisocyanate, cyclohexylene diisocyanate, isophorone diisocyanate, p-phenylene diisocyanate, toluene diisocyanate, isomers, trimers and higher oligomers of these diisocyanates, uretdiones of these isocyanates, cyanurates and isocyanurates of these isocyanates and mixtures of two or more thereof.

12. Formulation for the preparation of an open-cell polyurethane foam according to any one of the preceding claims, wherein the polyisocyanate component (b) is contained in an amount of 12 to 50% by weight.

13. Formulation for the preparation of an open-cell polyurethane foam according to claim 12, wherein the polyisocyanate component (b) is contained in an amount of 17 to 36% by weight.

14. Formulation for the preparation of an open-cell polyurethane foam according to any one of the preceding claims, wherein the formulation further comprises
(d) a catalyst component containing a basic compound or a Lewis acidic compound and/or
(e) a propellant component containing an organic solvent as a physical propellant, a solid or water as a chemical propellant or a combination thereof.

15. Formulation for the preparation of an open-cell polyurethane foam according to claim 14, wherein the catalyst component (d) is contained in an amount of 0.01 to 5% by weight.

16. Formulation for the preparation of an open-cell polyurethane foam according to claim 15, wherein the catalyst component (d) is contained in an amount of 0.1 to 1% by weight.

17. Formulation for the preparation of an open-cell polyurethane foam according to any one of claims 14 to 16, wherein the organic solvent of the propellant component (e) is selected from the group comprising optionally substituted straight-chained, branched-chained and cyclic alkanes, acetals, ketones, esters, halogenated hydrocarbons or mixtures thereof.

18. Formulation for the preparation of an open-cell polyurethane foam according to claim 17, wherein the organic solvent of the propellant component (e) is selected from the group comprising pentane, hexane, heptane, isooctane, cyclohexane, dimethoxy methane, 1,1-dimethoxy ethane, acetone, ethylacetate, chloroform, dichloromethane and dichloroethane.

19. Formulation for the preparation of an open-cell polyurethane foam according to any one of claims 14 to 18, wherein the solid of the propellant component (e) is an inorganic or organic compound selected from the group comprising ammonium carbonate, ammonium bicarbonate, ammonium oxalate, carbazides, hydrazides, azo compounds and diazo compounds.

20. Formulation for the preparation of an open-cell polyurethane foam according to any one of claims 14 to 19, wherein the organic solvent of the propellant component (e) is contained in an amount of 7 to 30% by weight, the solid propellant is contained in an amount of 1 to 5% by weight and the water of the propellant component is contained in an amount of 0.01 to 1% by weight.

21. Formulation for the preparation of an open-cell polyurethane foam according to any one of the preceding claims, wherein the formulation optionally contains one or more further ingredients selected from the group comprising diluents, plasticizers, surfactants, foam stabilizers, nucleating agents, compounds for adjusting the surface tension and the polarity, viscosity modifiers, silicone oils, inorganic particles or nanoparticles, sodium chloride and other salts as well as amino acids.

22. Open-cell polyurethane foam obtainable by heating a formulation according to any one of claims 1 to 21 to a temperature of about 30°C to about 90°C for a period of about 1 minute to about 24 hours.

23. Open-cell polyurethane foam according to claims 22, wherein the polyurethane foam is biocompatible and/or biodegradable.

24. Open-cell polyurethane foam according to claim 22 or 23, wherein the hydrophilicity of the polyurethane foam has been further increased by boiling in water for a period of about 1 min to about 24 h and/or has been adjusted by treating with alcohol, water or culture medium in a gradual transition.

25. Method for the preparation of an open-cell polyurethane foam according to any one of claims 22 to 24, wherein the components of the formulation according to any one of claims 1 to 21 are mixed and the mixture is heated for a period of about 3 min to about 24 h to a temperature of about 30°C to about 90°C.

26. Method according to claim 25, wherein the polyurethane foam is further boiled in water for a period of 1 min to 24 h.

27. Method according to claim 25 or 26, wherein the polyurethane foam has been further treated with alcohol, water or culture medium in a gradual transition.

28. Use of a open-cell polyurethane foam according to any one of claims 22 to 24 as a filter medium or a sponge.

29. Use of an open-cell polyurethane foam according to any one of claims 22 to 24 in medicine.

30. Use of an open-cell polyurethane foam according to any one of claims 22 to 24 as a support material for medicaments.

31. Use of an open-cell polyurethane foam according to any one of claims 22 to 24 as a support for cell cultures.

32. Use of an open-cell polyurethane foam according to any one of claims 22 to 24 as a support for tissue cultures.

33. Use of an open-cell polyurethane foam according to any one of claims 22 to 24 for the preparation of a scaffold for tissue engineering.

34. Use of an open-cell polyurethane foam according to claim 33, wherein the scaffold serves for the formation of implants as a replacement material for cartilages and in situations requiring cartilage-bone connections.

35. Use of an open-cell polyurethane foam according to claim 34, wherein the implants serve as a replacement material for cartilages in the region of the ears, the nose, the intervertebral discs or the meniscus.

36. Method for the preparation of a scaffold for tissue engineering made of an open-cell polyurethane foam according to any one of claims 22 to 24, comprising the steps of:
(a) recording the exterior form of a desired implant by an image forming method at a patient,
(b) preparing a negative mould of said implant as a casting mould from a suitable material using the data obtained in step (a),
(c) filling the negative mould with a formulation for the preparation of an open-cell polyurethane foam according to anyone of claims 1 to 21,
(d) curing the formulation,
(e) removing the negative mould to obtain the scaffold made of an open-cell polyurethane foam and
(f) conditioning the scaffold for the colonization with cells or cell-containing support media by boiling in water or by treating with alcohols, water or culture medium in a gradual transition.

37. Method according to claim 36, wherein the material of the casting mould in step (b) is a silicone.

38. Scaffold obtained by the method according to any one of claims 36 and 37.

## Revendications

1. Formulation pour la fabrication d'une mousse de polyuréthane à pores ouverts, qui ne présente pas de pellicule sur la face extérieure, comprenant:
(a) un composant polyol, contenant au moins un composé comprenant des groupes hydroxy, en une quantité de 15 à 85 % en poids,
(b) un composant polyisocyanate, contenant au moins un composé comprenant des groupes isocyanates, en une quantité de 8 à 70 % en poids, et
(c) un composant saccharide, contenant au moins un mono-, di-, oligo- ou polysaccharide, à l'exception de l'amidon, en une quantité de 0,01 à 4,20 % en poids, la quantité du composant saccharide atteignant moins de 5 % en poids par rapport au composant polyol (a).

2. Formulation pour la fabrication d'une mousse de polyuréthane à pores ouverts selon la revendication 1, dans laquelle le composant saccharide (c) comprend au moins un mono-, di-, oligo- ou polysaccharide, choisi dans le groupe comprenant le dextrose, le mannose, le mannite, le dulcite, le glucose, le fructose, le galactose, le maltose, le lactose, le saccharose, la cellobiose, la cellulose, la pectine, l'amylopectine et des mélanges de deux ou plusieurs de ceux-ci.

3. Formulation pour la fabrication d'une mousse de polyuréthane à pores ouverts selon l'une des revendications précédentes, dans laquelle le composant saccharide (c) est présent en une quantité de 0,5 à 3,70 % en poids.

4. Formulation pour la fabrication d'une mousse de polyuréthane à pores ouverts selon l'une des revendications précédentes, dans laquelle le composant saccharide (c) est présent en une quantité de 0,7 à 3 % en poids.

5. Formulation pour la fabrication d'une mousse de polyuréthane à pores ouverts selon l'une des revendications précédentes, dans laquelle le composant polyol (a) contient au moins un composé contenant des groupes hydroxy, choisi dans le groupe comprenant :
les polyéthers à terminaison hydroxy, les copolymères de polyéthers à terminaison hydroxy, les polycarbonates aliphatiques à terminaison hydroxy, les copolymères de polycarbonates aliphatiques à terminaison hydroxy, les polyanhydrides d'acides dicarboxyliques, les copolymères de ces polyanhydrides, les alcools divalents ou multivalents de faible poids moléculaire, les dimères, trimères ou oligomères d'acides aminés contenant des groupes hydroxy, les alcools de sucre, d'autres substances naturelles ou dérivés de substances naturelles ayant au moins deux groupes hydroxy, les polyesters dont les groupes terminaux sont des groupes hydroxy, ainsi que les copolymères des polyesters de ce type, les copolymères à blocs segmentés constitués de segments de polyéthers et de polyesters et des mélanges de deux ou plusieurs de ceux-ci.

6. Formulation pour la fabrication d'une mousse de polyuréthane à pores ouverts selon la revendication 5, dans laquelle le composant polyol (a) comprend au moins un composé contenant des groupes hydroxy choisi dans le groupe comprenant:
l'α,ω-dihydroxypoly(oxyéthylène), l'α,ω-dihydroxypoly(1,2-éthylène oxyde), l'α,ω-dihydroxypoly(1,2-propylène oxyde), l'α,ω-dihydroxypoly(1,3-triméthylène oxyde), l'α,ω-dihydroxypoly(1,4-tétraméthylène oxyde), l'α,ω-dihydroxypoly(méthylènoxy-1,2-éthylène oxyde) ainsi que leurs copolymères, respectivement avec des masses molaires allant jusqu'à 15 000 g/mol, l'α,ω-dihydroxypoly(éthylène carbonate), l'α,ω-dihydroxypoly(1,2-propylène carbonate), l'α,ω-dihydroxypoly(1,3-propylène carbonate), l'α,ω-dihydroxypoly(tétraméthylène carbonate), l'α,ω-dihydroxypoly(hexaméthylène carbonate), leurs copolymères, respectivement avec des masses molaires allant jusqu'à 15000 g/mol, les polyanhydrides de l'acide malonique, de l'acide succinique, de l'acide glutarique ou de leurs mélanges, ainsi que leurs copolymères, respectivement avec des masses molaires allant jusqu'à 15 000 g/mol, le polycaprolactonediol ayant un poids moléculaire moyen en nombre allant jusqu'à 15000 g/mol, le polycaprolactonetriol ayant un poids moléculaire moyen en nombre allant jusqu'à 15000 g/mol, le glycol, le 1,2-propylène glycol, le 1,3-propylène glycol, le butanediol, le pentanediol, l'hexanediol, la glycérine, la triéthanolamine, le pentaérythrite, le 2,2-bis(hydroxyméthyl)propanol, les trimères ou oligomères d'acides aminés contenant des groupes hydroxy issus de la tyrosine et/ou de la sérine, ainsi que le sorbite et des mélanges de deux ou plusieurs de ceux-ci.

7. Formulation pour la fabrication d'une mousse de polyuréthane à pores ouverts selon la revendication 5, dans laquelle le composant polyol (a) comprend au moins un composé contenant des groupes hydroxy choisi dans le groupe comprenant le polycaprolactonediol ayant un poids moléculaire moyen en nombre allant de 200 g/mol à 5000 g/mol, le polycaprolactonetriol ayant un poids moléculaire moyen en nombre allant de 200 g/mol à 5000 g/mol et leurs mélanges.

8. Formulation pour la fabrication d'une mousse de polyuréthane à pores ouverts selon l'une des revendications précédentes, dans laquelle le composant polyol (a) est présent en une quantité de 30 à 80 % en poids.

9. Formulation pour la fabrication d'une mousse de polyuréthane à pores ouverts selon la revendication 8, dans laquelle le composant polyol (a) est présent en une quantité de 45 à 75 % en poids.

10. Formulation pour la fabrication d'une mousse de polyuréthane à pores ouverts selon l'une des revendications précédentes, dans laquelle le composant polyisocyanate (b) comprend au moins un composé contenant des groupes isocyanates, choisi dans le groupe comprenant :
les diisocyanates d'alkylène le cas échéant substitués ayant de 3 à 12 atomes de carbone, les diisocyanates de cycloalkylène le cas échéant substitués ayant de 5 à 15 atomes de carbone, les diisocyanates d'alkylcycloalkylène le cas échéant substitués ayant de 6 à 18 atomes de carbone, les diisocyanates aromatiques le cas échéant substitués, les trimères et oligomères supérieurs de ces diisocyanates, les urètediones de ces isocyanates, les cyanurates et isocyanurates de ces isocyanates et les mélanges de deux ou plusieurs de ceux-ci.

11. Formulation pour la fabrication d'une mousse de polyuréthane à pores ouverts selon la revendication 10, dans laquelle le composant polyisocyanate (b) comprend au moins un composé contenant des groupes isocyanates, choisi dans le groupe comprenant:
le diisocyanate d'hexaméthylène, le diisocyanate de tétraméthylène, le diisocyanate de lysine, le diisocyanate de cyclohexylène, le diisocyanate d'isophorone, le diisocyanate de p-phénylène, le diisocyanate de toluyle, les isomères, trimères et oligomères supérieurs de ces diisocyanates, les urètediones de ces isocyanates, les cyanurates et isocyanurates de ces isocyanates et les mélanges de deux ou plusieurs de ceux-ci.

12. Formulation pour la fabrication d'une mousse de polyuréthane à pores ouverts selon l'une des revendications précédentes, dans laquelle le composant polyisocyanate (b) est présent en une quantité de 12 à 50 % en poids.

13. Formulation pour la fabrication d'une mousse de polyuréthane à pores ouverts selon la revendication 12, dans laquelle le composant polyisocyanate (b) est présent en une quantité de 17 à 36 % en poids.

14. Formulation pour la fabrication d'une mousse de polyuréthane à pores ouverts selon l'une des revendications précédentes, dans laquelle la formulation comprend, en plus
(d) un composant catalytique, qui contient un composé de base ou d'acide de Lewis, et/ou
(e) un composant propulseur, qui contient un solvant organique comme propulseur physique, une matière solide ou de l'eau comme propulseur chimique ou une combinaison des deux.

15. Formulation pour la fabrication d'une mousse de polyuréthane à pores ouverts selon la revendication 14, dans laquelle le composant catalytique (d) est présent en une quantité allant de 0,01 à 5 % en poids.

16. Formulation pour la fabrication d'une mousse de polyuréthane à pores ouverts selon la revendication 15, dans laquelle le composant catalytique (d) est présent en une quantité allant de 0,01 à 1 % en poids.

17. Formulation pour la fabrication d'une mousse de polyuréthane à pores ouverts selon l'une des revendications 14 à 16, dans laquelle le solvant organique du composant propulseur (e) est choisi dans le groupe comprenant les alcanes cycliques et linéaires, ramifiés le cas échéant substitués, les acétals, les cétones, les esters, les hydrocarbures halogénés ou mélanges de ceux-ci.

18. Formulation pour la fabrication d'une mousse de polyuréthane à pores ouverts selon la revendication 17, dans laquelle le solvant organique du composant propulseur (e) est choisi dans le groupe comprenant le pentane, l'hexane, l'heptane, l'isooctane, le cyclohexane, le diméthoxyméthane, le 1,1-diméthoxyéthane, l'acétone, l'acétate d'éthyle, le chloroforme, le dichlorométhane et le dichloroéthane.

19. Formulation pour la fabrication d'une mousse de polyuréthane à pores ouverts selon l'une des revendications 14 à 18, dans laquelle la matière solide du composant propulseur (e) est un composé inorganique ou organique, choisi dans le groupe comprenant le carbonate d'ammonium, l'hydrogéno-carbonate d'ammonium, l'oxalate d'ammonium, les carbazides, les hydrazides, les composés azoïques et les composés diazoïques.

20. Formulation pour la fabrication d'une mousse de polyuréthane à pores ouverts selon l'une des revendications 14 à 19, dans laquelle le solvant organique du composant propulseur (e) est présent en une quantité de 7 à 30 % en poids, le propulseur solide en une quantité de 1 à 5 % en poids et l'eau du composant propulseur en une quantité de 0,01 à 1 % en poids.

21. Formulation pour la fabrication d'une mousse de polyuréthane à pores ouverts selon l'une des revendications précédentes, dans laquelle la formulation comprend le cas échéant un ou plusieurs autres ingrédients, choisis dans le groupe comprenant les diluants, les plastifiants, les tensioactifs, les stabilisateurs de mousse, les agents de nucléation, les composés régulant la tension de surface et la polarité, les améliorants de viscosité, les huiles siliconées, les particules inorganiques ou les nanoparticules, le sel de cuisine et autres sels ainsi que les acides aminés.

22. Mousse de polyuréthane à pores ouverts, pouvant être obtenue par chauffage d'une formulation selon l'une des revendications 1 à 21 à une température d'environ 30°C à environ 90°C pendant une durée d'environ 1 min à environ 24 h.

23. Mousse de polyuréthane à pores ouverts selon la revendication 22, dans laquelle la mousse de polyuréthane est biocompatible et/ou biodégradable.

24. Mousse de polyuréthane à pores ouverts selon la revendication 22 ou 23, dans laquelle l'hydrophilie de la mousse de polyuréthane a été encore augmentée par ébullition dans l'eau pendant une durée d'environ 1 min à environ 24 h et/ou a été réglée par traitement avec de l'alcool, de l'eau ou un milieu de culture dans une transition par étapes.

25. Procédé de fabrication d'une mousse de polyuréthane à pores ouverts selon l'une des revendications 22 à 24, dans lequel les composants de la formulation selon l'une des revendications 1 à 21 sont mélangés ensemble et le mélange est chauffé pendant une durée d'environ 3 min à environ 24 h à une température d'environ 30°C à environ 90°C.

26. Procédé selon la revendication 25, dans lequel la mousse de polyuréthane est en outre bouillie dans l'eau pendant une durée de 1 min à 24 h.

27. Procédé selon la revendication 25 ou la revendication 26, dans lequel la mousse de polyuréthane a été en outre traitée dans une transition par étapes avec de l'alcool, de l'eau ou un milieu de culture.

28. Utilisation d'une mousse de polyuréthane à pores ouverts selon l'une des revendications 22 à 24 à titre de filtre ou d'éponge.

29. Utilisation d'une mousse de polyuréthane à pores ouverts selon l'une des revendications 22 à 24 en médecine.

30. Utilisation d'une mousse de polyuréthane à pores ouverts selon l'une des revendications 22 à 24 comme matériau support pour des médicaments.

31. Utilisation d'une mousse de polyuréthane à pores ouverts selon l'une des revendications 22 à 24 comme supports pour des cultures cellulaires.

32. Utilisation d'une mousse de polyuréthane à pores ouverts selon l'une des revendications 22 à 24 comme supports pour des cultures tissulaires.

33. Utilisation d'une mousse de polyuréthane à pores ouverts selon l'une des revendications 22 à 24 pour la fabrication d'un support cellulaire pour le génie tissulaire.

34. Utilisation d'une mousse de polyuréthane à pores ouverts selon la revendication 33, dans laquelle le support cellulaire sert à construire des implants à titre de remplacement pour le cartilage et dans des situations, où des liaisons cartilagino-osseuses sont nécessaires.

35. Utilisation d'une mousse de polyuréthane à pores ouverts selon la revendication 34, dans laquelle les implants servent de remplacement pour le cartilage dans la zone des oreilles, du nez, des disques intervertébraux ou du ménisque.

36. Procédé de fabrication d'un support cellulaire pour le génie tissulaire constitué d'une mousse de polyuréthane à pores ouverts selon l'une des revendications 22 à 24, qui comprend les étapes suivantes:
(a) prise de la forme extérieure d'un implant voulu grâce à un procédé de prise d'empreinte chez un patient,
(b) élaboration d'un modèle en négatif de cet implant sous forme de moule de coulée constitué d'un matériau approprié en utilisant les données obtenues à l'étape (a),
(c) remplissage du modèle en négatif avec une formulation pour la fabrication d'une mousse de polyuréthane à pores ouverts selon l'une des revendications 1 à 21,
(d) durcissement de la formulation et
(e) élimination du modèle en négatif en obtenant le support cellulaire constitué d'une mousse de polyuréthane à pores ouverts
(f) conditionnement pour la colonisation par des cellules ou des milieux supports contenant des cellules par ébullition dans de l'eau ou par traitement avec de l'alcool, de l'eau ou un milieu de culture dans une transition par étapes.

37. Procédé selon la revendication 36, dans lequel le matériau du moule de coulée à l'étape (b) est la silicone.

38. Support cellulaire, obtenu par le procédé selon l'une des revendications 36 et 37.
